# EUROPÄISCHE PATENTANMELDUNG

(11) **EP 2 724 870 A1**
(43) Veröffentlichungstag der Anmeldung: **30.04.2014**
(21) Anmeldenummer: 12189995.9
(22) Anmeldetag: 25.10.2012
(51) Int. Cl.: B60C 1/00, C08L 21/00, C08K 5/372, C07C 321/12

(54) **Polysulfidmischungen, Verfahren zu ihrer Herstellung und Verwendung der Polysulfidmischungen in Kautschukmischungen**

(71) Anmelder: LANXESS Deutschland GmbH, 50569 Köln (DE)
(72) Erfinder: Feldhues, Ulrich, 51465 Bergisch Gladbach (DE); Unterberg, Heinz, 41540 Dormagen (DE); Weidenhaupt, Hermann-Josef, 50259 Pulheim (DE); Wiedemeier-Jarad, Melanie, 41540 Dormagen (DE)

(57) **Zusammenfassung**

Die vorliegende Erfindung betrifft neue Polysulfidmischungen enthaltend Verbindungen der Formel (I) 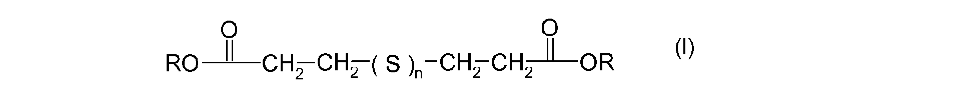 wobei n für 2 bis 6 und R für C₁-C₈-Alkyl steht, Verfahren zur Herstellung dieser Polysulfidmischungen, deren Verwendung zur Herstellung von Kautschukmischungen und daraus hergestellte Kautschukvulkanisate.

## Beschreibung

Die vorliegende Erfindung betrifft neue Polysulfidmischungen, Verfahren zur Herstellung dieser Polysulfidmischungen, die Verwendung der Polysulfidmischungen in Kautschukmischungen, daraus hergestellte Kautschukvulkanisate und deren Verwendung.

Kieselsäurehaltige Kautschukmischungen sind wichtige Ausgangsmaterialien, beispielsweise für die Herstellung von Reifen mit verringertem Rollwiderstand. Diese leisten beim Abrollen weniger Verformungsarbeit und senken deshalb den Kraftstoffverbrauch. Durch die in verschiedenen Staaten beschlossene Kennzeichnungspflicht des Rollwiderstands für Reifen besteht ein großes Interesse daran, diesen Widerstand weiter zu senken.

Zur Verringerung des Rollwiderstands sind eine Reihe von Lösungsvorschlägen ausgearbeitet worden. In DE 2 255 577 A und DE 4 435 311 A, EP-A 0 0670 347 sowie US-A-4 709 065 sind polysulfidische Silane als Verstärkungsadditive für kieselsäurehaltige Kautschukvulkanisate beschrieben. Das Eigenschaftsprofil der so hergestellten Kautschukvulkanisate ist noch nicht optimal. Insbesondere die Abriebwerte sind unvorteilhaft hoch. Hohe Abriebwerte können sich sehr nachteilig auf die Lebensdauer der Produkte auswirken.

Bei der Verarbeitung von Kautschuken ist es vorteilhaft, wenn die zunächst zubereitete Kautschukmischung mit den Additiven eine niedrige Fließviskosität (Mooney Viskosität ML 1+4/100°C) aufweist und dadurch leicht verarbeitet werden kann. Zur Verbesserung der Verarbeitbarkeit von kieselsäurehaltigen Kautschukmischungen wurden weitere Zusatzstoffe vorgeschlagen, wie Fettsäureester, Fettsäuresalze oder Mineralöle. Die genannten Zusatzstoffe besitzen den Nachteil, dass sie die Fließfähigkeit erhöhen, gleichzeitig aber die Spannungswerte bei größerer Dehnung (z.B. 100% bis 300%) oder auch die Härte der Vulkanisate stark vermindern, so dass die verstärkende Wirkung des Füllstoffes Einbuße erleidet. Eine ungenügende Härte oder Steifigkeit des Vulkanisats resultiert aber in einem unbefriedigenden Fahrverhalten des Reifens besonders in Kurven. Zur Erhöhung der Härte des Vulkanisats kann der Anteil an verstärkendem Füllstoff erhöht, oder der Anteil an Weichmacheröl verringert werden, wobei jede dieser beiden Maßnahmen den Nachteil der höheren Mischungsviskosität bei der Verarbeitung bedingt.

In der EP 0 489 313 werden Glykolfunktionen enthaltende Additive mit guten mechanischen Eigenschaften und verbessertem Hystereseverhalten beschrieben. Im Vergleich mit dem Bis-[-3-(triethoxysilyl)-propyl]-tetrasulfid gemäß DE-OS 2 255 577 zeigen die Beispiele aber keine Verbesserung im Rollwiderstand (tan δ bei 60°C).

Die Herstellung einer Polysulfidmischung auf Basis der Formel (I) durch Rhodiumkatalysierten Schwefelaustausch wurde von Arisawa et. al in Tetrahedron Letters, 2005, Volume 46, Ausgabe 28, Seiten 4797-4800 beschrieben.

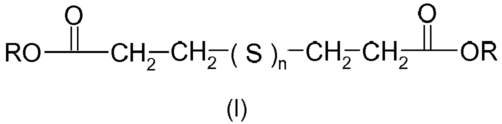

Die offenbarte Polysulfidmischung enthält Polysulfide der Formeln (II), (III), (IV), (V) und (VI):

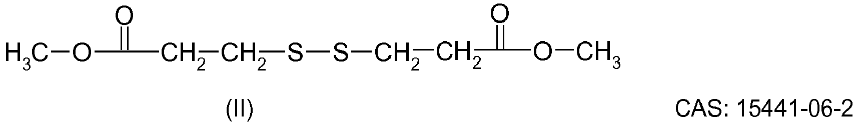

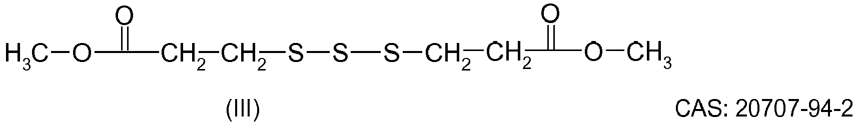

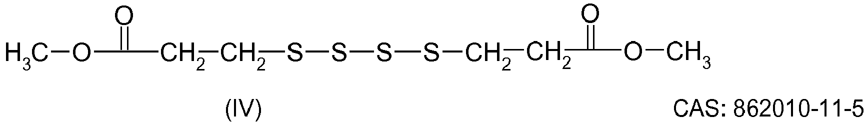

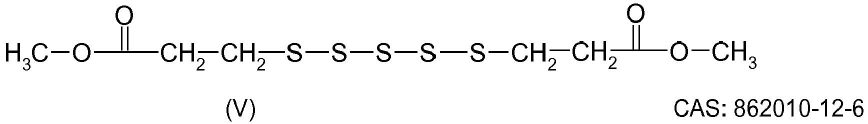

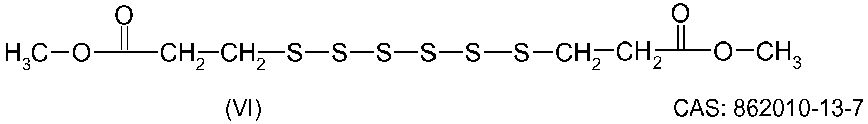

Die von Arisawa et. al offenbarte Polysulfidmischung enthält ca.

| in Mol% | in Gew.% | |
|---|---|---|
| - 49% | 44% | Verbindung (II) |
| - 26% | 26% | Verbindung (III) |
| - 14% | 16% | Verbindung (IV) |
| - 7% | 9% | Verbindung (V) |
| - 4% | 5% | Verbindung (VI). |

Eine praktische Anwendung der offenbarten Polysulfidmischung ist nicht beschrieben.

Einer Verwendung in Kautschukmischungen steht auch der hohe Anteil an Verbindung (II) entgegen. Diese hat ein relativ niedriges Molekulargewicht (238,3), einen relativ niedrigen Flammpunkt (150°C) und einen intensiven unangenehmen Geruch. Weiterhin enthält sie nur Schwefelatome, die an mindestens ein Kohlenstoffatom gebunden sind und keine Schwefelatome, die nur an Schwefelatome gebunden sind, damit für Reaktionen leichter zur Verfügung stehen und nachfolgend als aktive Schwefelatome bezeichnet werden. Somit enthält die von Arisawa et. al offenbarte Mischung im Durchschnitt weniger als drei Schwefelatome pro Molekül und damit im Durchschnitt weniger als ein aktives mittleres Schwefelatom pro Molekül.

In der bis dato unveröffentlichten Anmeldung EP 11164319 ist die Verwendung eines Polysulfids der Formel (IV) mit R = Methyl in kieselsäurehaltigen Kautschukmischungen beschrieben. Die Nachstellung des offenbarten Versuchs zeigte, dass in EP 11164319 als Polysulfid eine Mischung eingesetzt wurde, welche 65% der Verbindung (IV) und 35% der Verbindungen (II), (III), (V) und (VI), insbesondere 31% der Verbindungen (III) und (V) enthielt. Durch die Verwendung dieser Polysulfidmischung wurde zwar eine Verbesserung der Mooney-Viskosität und Härte erzielt, allerdings auf Kosten eines deutlich erhöhten Rollwiderstands und Abriebs, weshalb das Polysulfid für die Verwendung in Reifen nicht optimal ist.

Aufgabe der vorliegenden Erfindung ist es, die genannten Nachteile des Stands der Technik zu beseitigen und neue verbesserte Polysulfidmischungen und Verfahren zu ihrer Herstellung zu finden und damit neue Kautschukmischungen bereitzustellen, die bei weiterhin guter Fließfähigkeit in Vulkanisate überführt werden können, welche einen signifikant verbesserten Rollwiderstand in Verbindung mit hoher Härte und geringem Abrieb aufweisen.

Basierend auf der Erkenntnis, dass Verbindungen der Formel (IV) bei Erwärmung über 45°C leicht zu Verbindungen mit höherer und niederer Anzahl Schwefelatome disproportionieren, wurde nun ein Verfahren entwickelt, welches erfindungsgemäße Polysulfidmischungen liefert, die bedingt durch eine enge Verteilung der Länge der Schwefelketten in der Mischung, überraschenderweise die Aufgabe der Erfindung zu lösen vermögen.

Das erfindungsgemäße Verfahren zur Herstellung von Polysulfidmischungen umfasst das Inkontaktbringen von 3-Mercaptopropionsäurealkylestern (VII), deren Herstellung in einer Vielzahl von Literaturstellen beschrieben ist, mit S₂Cl₂, 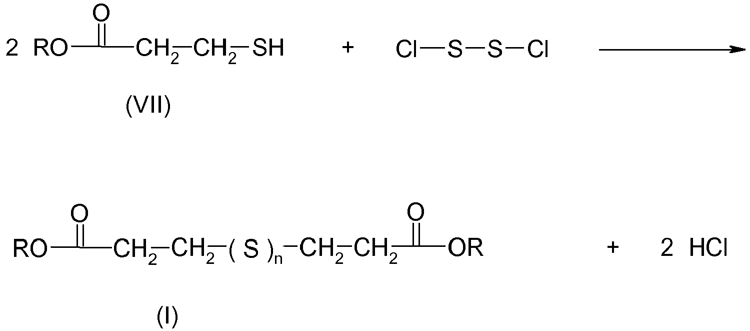 wobei jeder der beiden Reste R identisch oder verschieden, vorzugsweise identisch ist und für Methyl, Ethyl, n-Propyl, 2-Propyl, n-Butyl, Isobutyl, t-Butyl oder C₅-Alkyl, C₆-Alkyl, C₇-Alkyl oder C₈-Alkyl vorzugsweise Methyl, Ethyl, n-Propyl, n-Butyl oder Isooctyl, besonders bevorzugt Methyl steht und n eine Zahl von 2 bis 6 ist.

Prinzipiell können auch verschiedene Mercaptopropionsäurealkylester (VII) eingesetzt werden, wodurch Polysulfidmischungen enstehen, welche asymmetrische Polysulfide enthalten, d.h. Polysulfide die zwei unterschiedliche Substituenten R tragen. Solche Mischungen sind im Rahmen der vorliegenden Erfindung als äquivalent zu den Mischungen aus Polysulfiden mit gleichen Substituenten R zu betrachten. Bei der bevorzugten Ausführungsform der vorliegenden Erfindung handelt es sich um eine Polysulfidmischung aus symmetrischen Polysulfiden, d.h. Polysulfide die zwei gleiche Substituenten tragen, insbesondere Mischungen, bei denen R bei allen Polysulfiden gleich ist.

Das Inkontaktbringen erfolgt typischerweise in einem inerten Medium, worunter ein Medium verstanden wird, welches unter den Reaktionsbedingungen mit max. 10%, vorzugsweise max. 5% besonders bevorzugt max. 1%, ganz besonders bevorzugt überhaupt nicht mit den Reaktionskomponenten reagiert. Als solches Medium sind vorzugsweise unter Reaktionsbedingungen flüssige aliphatische cyclische und/oder acyclische Kohlenwasserstoffe, aromatische Kohlenwasserstoffe, aliphatische und/oder aromatische Halogenkohlenwasserstoffe, Ether und Ester geeignet und Gemische davon geeignet. Besonders bevorzugte Medien sind Toluol und Cyclohexan. Vorzugsweise werden wasserfreie Medien verwendet.

In einer bevorzugten Ausführungsform erfolgt das Inkontaktbringen in einer Mischung aus Polysulfiden der Formel (I), insbesondere eine der zuvor beschriebenen erfindungsgemäßen Polysulfidmischungen, wodurch auf die Abtrennung des inerten Mediums verzichtet werden kann.

Vorzugsweise wird eine Mischung von 3-Mercaptopropionsäurealkylester (VII) und inertem Medium vorgelegt und dann S₂Cl₂ unter Kühlung des Reaktionsansatzes zudosiert. Die Edukte können aber auch gleichzeitig in das inerte Medium eingetragen werden. Das Verfahren kann sowohl in Batch-Fahrweise, in kontinuierlicher, halbkontinuierlicher oder in Kaskadenfahrweise durchgeführt werden.

Das Inkontaktbringen erfolgt typischerweise bei Temperaturen von 0°C bis 60°C, bevorzugt von 10 °C bis 45 °C, besonders bevorzugt von 15°C bis 35°C. Bei zu niedrigen Temperaturen reagiert S₂Cl₂ ggf. nicht sofort komplett ab, was zu einem Gefährdungspotential und Nebenprodukten durch den Aufbau einer erhöhten S₂Cl₂-Konzentration im Reaktionsansatz führen kann. Zu hohe Temperaturen sind aus Gründen von Arbeitssicherheit und Produktqualität ebenfalls zu vermeiden.

Vorzugsweise wird die Reaktion unter Inertgas durchgeführt. Hierfür werden beispielsweise Edelgase oder Stickstoff verwendet.

Bevorzugt wird das entstehende HCl-Gas bereits während der Reaktion ganz oder anteilig aus dem Ansatz entfernt, insbesondere durch Durchleiten von Inertgas durch den Reaktionsansatz und/oder durch Anlegen von Vakuum, d. h. einem Druck von weniger als 1013 mbar.

Falls als inertes Medium eine erfindungsgemäße Polysulfidmischung verwendet wird, so entfällt die Abtrennung des inerten Mediums. Andernfalls, d. h. wenn ein anderes inertes Medium als eine erfindungsgemäße Polysulfidmischung verwendet wird, so erfolgt die Abtrennung dieses inerten Mediums bei einer Temperatur von nicht mehr als 45 °C, bevorzugt bei nicht mehr als 40 °C, besonders bevorzugt bei nicht mehr als 35 °C und ganz besonders bevorzugt nicht mehr als 30 °C und wird vorzugsweise bei vermindertem Druck, d.h. weniger als 1013 mbar durchgeführt.

In einer bevorzugten Ausführungsform erfolgt somit sowohl das Inkontaktbringen von 3-Mercaptopropionsäurealkylestern (VII) mit S₂Cl₂ als auch die Abtrennung von einem inerten Medium, sofern es sich dabei nicht um eine erfindungsgemäße Polysulfidmischung handelt, im Temperaturbereich von 10 °C bis 45 °C, besonders bevorzugt von 15°C bis 35°C, ganz besonders bevorzugt von 20 °C bis 30 °C.

Die vorliegende Erfindung betrifft neben dem Verfahren zur Herstellung von erfindungsgemäßen Polysulfidmischungen auch die dadurch erhaltenen Polysulfidmischungen der Formel (I), wobei jeder der beiden Reste R identisch oder verschieden, vorzugsweise identisch ist und für Methyl, Ethyl, n-Propyl, 2-Propyl, n-Butyl, Isobutyl, t-Butyl oder C₅-Alkyl, C₆-Alkyl, C₇-Alkyl oder C₈-Alkyl vorzugsweise Methyl, Ethyl, n-Propyl, n-Butyl oder Isooctyl, besonders bevorzugt Methyl steht und n eine Zahl von 2 bis 6 ist.

Die erfindungsgemäßen Polysulfidmischungen enthalten mindestens 80%, bevorzugt mindestens 85%, besonders bevorzugt mindestens 90% und ganz besonders bevorzugt 95 bis 99% einer oder mehrerer Polysulfidverbindungen der Formel (I) mit n = 4 bezogen auf die Gesamtmenge an Polysulfidverbindungen der Formel (I). Im Rahmen der vorliegenden Anmeldung sind die Mengenangaben in Bezug auf die Verbindungen der Formel (I) als Flächenprozentangaben bei der HPLC-Messung mit Brechungsindex (RI)-Detektor zu verstehen, wie sie im Anschluss an Beispiel 3 beschrieben ist.

Üblicherweise enthält die erfindungsgemäße Polysulfidmischung weniger als 10%, bevorzugt weniger als 6%, besonders bevorzugt weniger als 4% und ganz besonders bevorzugt weniger als 2% an Polysulfidverbindungen der Formel (I) mit n = 3 bezogen auf die Gesamtmenge an Polysulfidverbindungen der Formel (I) mit.

Üblicherweise enthält die erfindungsgemäße Polysulfidmischung weniger als 10%, bevorzugt weniger als 6%, besonders bevorzugt weniger als 4% und ganz besonders bevorzugt weniger als 3% an Polysulfidverbindungen der Formel (I) mit n = 5 bezogen auf die Gesamtmenge an Polysulfidverbindungen der Formel (I).

Üblicherweise enthält die erfindungsgemäße Polysulfidmischung weniger als 10%, bevorzugt weniger als 5%, besonders bevorzugt weniger als 2% und ganz besonders bevorzugt weniger als 1% an Polysulfidverbindungen der Formel (I) mit n = 2 bezogen auf die Gesamtmenge an Polysulfidverbindungen der Formel (I).

Üblicherweise enthält die erfindungsgemäße Polysulfidmischung weniger als 3%, bevorzugt weniger als 2% und ganz besonders bevorzugt nicht mehr als 1% an Polysulfidverbindungen der Formel (I) mit n = 6 bezogen auf die Gesamtmenge an Polysulfidverbindungen der Formel (I).

Die erfindungsgemäße Polysulfidmischung enthält neben einer oder mehrerer Verbindungen der Formel (I) mit n = 4 noch eine oder mehrere Verbindungen der Formel (I) mit n = 2, 3, 5 oder 6. Üblicherweise beträgt der aufsummierte Anteil der Verbindungen der Formel (I) mit n = 2, 3, 5 und 6 nicht mehr als 20%, vorzugsweise nicht mehr als 15 %, besonders bevorzugt nicht mehr als 10 % und ganz besonders bevorzugt 1 bis 5% bezogen auf die Gesamtmenge an Polysulfidverbindungen der Formel (I), in der entsprechenden Mischung.

In einer bevorzugten Ausführungsform enthält die erfindungsgemäße Polysulfidmischung weniger als 10% besonders bevorzugt weniger als 3%, ganz besonders bevorzugt weniger als 1% und meist bevorzugt weniger als 0,2% Nebenprodukte oder Beimischungen, also Verbindungen, die nicht der Formel (I) entsprechen.

Typischerweise enthält die erfindungsgemäße Polysulfidmischung weniger als 2%, bevorzugt weniger als 1%, besonders bevorzugt weniger als 0,3%, ganz besonders bevorzugt weniger 0,1% und meist bevorzugt keinen elementaren Schwefel als Beimischung.

Eine erfindungsgemäße Polysulfidmischung weist üblicherweise einen Flammpunkt größer als 180°C, bevorzugt größer als 200°C auf.

Der Schwefelgehalt der erfindungsgemäßen Polysulfidmischung beträgt gewöhnlich gemäß Elementaranalyse 35 bis 50%, bevorzugt 40 bis 45%.

Eine erfindungsgemäße Polysulfidmischung hat typischerweise einen Gesamt-ChlorGehalt < 1000ppm, bevorzugt < 100ppm, besonders bevorzugt < 10ppm.

Üblicherweise hat die erfindungsgemäße Polysulfidmischung im Durchschnitt 3,5 - 4,5 Schwefelatome, bevorzugt 3,7 - 4,3 Schwefelatome, besonders bevorzugt 3,8 - 4,2 Schwefelatome, ganz besonders bevorzugt 3,9 - 4,1 Schwefelatome pro Molekül der Formel (I). Dadurch hat die erfindungsgemäße Polysulfidmischung typischerweise im Durchschnitt 1,5 - 2,5, bevorzugt 1,7 - 2,3, besonders bevorzugt 1,8 - 2,2, ganz besonders bevorzugt 1,9-2,1 aktive Schwefelatome pro Molekül der Formel (I).

Die erfindungsgemäßen Polysulfidmischungen der Formel (I) werden nach der Herstellung bevorzugt bei Temperaturen von 0 bis 35°C, insbesondere von 0 bis 20°C gelagert.

Die erfindungsgemäßen Polysulfidmischungen zeigen bei Anwendung in Kautschukmischungen unerwartete Eigenschaften. Gegenüber einer Referenzmischung ohne die erfindungsgemäße Polysulfidmischung wurden die Fließfähigkeit und Anvulkanisationszeit der Mischung, sowie die Härte und der Rollwiderstand des Vulkanisats verbessert. Dies ist umso erstaunlicher, als die in EP 11164319 verwendete Mischung enthaltend 65% der Verbindung (IV) und 31% der Verbindungen (III) und (V), d.h. einen vergleichbaren Gehalt an aktiven Schwefelatomen, eine deutliche Verschlechterung des Rollwiderstands und Abriebs gegenüber der Referenzmischung bewirkt.

Gegenstand der Erfindung sind somit auch Kautschukmischungen, enthaltend jeweils mindestens einen Kautschuk und eine erfindungsgemäße Polysulfidmischung.

Gegenstand der Erfindung sind insbesondere Kautschukmischungen, enthaltend jeweils mindestens einen Kautschuk, ein schwefelhaltiges Alkoxysilan, einen kieselsäurebasierenden Füllstoff, und eine erfindungsgemäße Polysulfidmischung.

Die erfindungsgemäßen Polysulfidmischungen können auch teilweise oder vollständig auf inerten, organischen oder anorganischen Trägern absorbiert eingesetzt werden. Bevorzugte Trägermaterialien sind Kieselsäure, natürliche und synthetische Silikate, Aluminiumoxid und/oder Russe.

Erfindungsgemäß beträgt der Gesamtgehalt an erfindungsgemäßer Polysulfidmischung in Kautschukmischungen vorzugsweise 0,1 bis 15 phr, besonders bevorzugt 0,3 bis 7 phr, ganz besonders bevorzugt 0,5 bis 3 phr und am meisten bevorzugt 0,7 bis 1,5 phr. Die Einheit phr steht für Gewichtsteile bezogen auf 100 Gewichtsteile an in der Kautschukmischung eingesetztem Kautschuk.

Für die Herstellung der erfindungsgemäßen Kautschukmischungen können Naturkautschuk und/oder Synthesekautschuke verwendet werden. Bevorzugte Synthesekautschuke sind z.B.:
- BR: Polybutadien
- ABR: Butadien/Acrylsäure-C₁-C₄-alkylester-Copolymerisat
- CR: Polychloropren
- IR: Polyisopren
- SBR: Styrol/Butadien-Copolymerisate mit Styrolgehalten von 1 bis 60, bevorzugt 20 bis 50 Gew.-%
- IIR: Isobutylen/Isopren-Copolymerisate
- NBR: Butadien/Acrylnitril-Copolymerisate mit 5-60, vorzugsw. 10-50 Gew-% Acrylnitrilgehalt
- HNBR: teilhydrierter oder vollständig hydrierter NBR-Kautschuk
- EPDM: Ethylen/Propylen/Dien-Copolymerisate
sowie Mischungen aus zwei oder mehr dieser Kautschuke.

Bevorzugt enthalten die erfindungsgemäßen Kautschukmischungen mindestens einen SBR-Kautschuk und mindestens einen BR-Kautschuk, besonders bevorzugt im Gewichtsverhältnis SBR:BR von 60:40 bis 90:10.

In einer vorteilhaften Ausführungsform enthalten die erfindungsgemäßen Kautschukmischungen zudem mindestens einen NR-Kautschuk. Besonders bevorzugt weisen sie wenigstens einen SBR-Kautschuk, wenigstens einen BR-Kautschuk und wenigstens einen NR-Kautschuk auf, wobei ganz besonders bevorzugt das Gewichtsverhältnis von SBR-Kautschuk zu BR-Kautschuk zu NR-Kautschuk 60 bis 85:10 bis 35:5 bis 20 beträgt.

Als schwefelhaltige Alkoxysilane für die erfindungsgemäßen Kautschukmischungen sind z.B. Bis-(triethoxysilylpropyl)tetrasulfan (z.B. Si 69 von Evonik) und Bis-(triethoxysilyl-propyl)disulfan (z.B. Si 75 von Evonik), 3-(Triethoxysilyl)-1-propanthiol, polyetherfunktionalisierte Mercaptosilane wie Si 363 von Evonik, thioesterfunktionalisierte Alkoxysilane wie NXT oder NXT Z von Momentive (früher GE) geeignet. Es können auch Mischungen der schwefelhaltigen Alkoxysilane eingesetzt werden. Flüssige schwefelhaltige Alkoxysilane können zur besseren Dosierbarkeit und/oder Dispergierbarkeit auf einem Träger aufgezogen sein (dry liquid). Der Wirkstoffgehalt liegt zwischen 30 und 70 Gew.-Teilen, bevorzugt 40 und 60 Gew.-Teilen je 100 Gew.-Teile dry liquid.

Der Anteil der schwefelhaltigen Alkoxysilane in den erfindungsgemäßen Kautschukmischungen beträgt vorzugsweise 2 bis 20 phr, besonders bevorzugt 3 bis 11 phr und ganz besonders bevorzugt 5 bis 8 phr, jeweils berechnet als 100%iger Wirkstoff. Vorzugsweise ist die Menge an schwefelhaltigem Alkoxysilan größer oder gleich der Menge an der erfindungsgemäßen Polysulfidmischung der Formel (I). Besonders bevorzugt beträgt das Gewichtsverhältnis von schwefelhaltigem Alkoxysilan zur erfindungsgemäßen Polysulfidmischung der Formel (I) 1,5:1 bis 20:1, ganz besonders bevorzugt 3:1 bis 15:1 und am meisten bevorzugt 5:1 bis 10:1.

Die erfindungsgemäß bevorzugte Kautschukmischung enthält zudem einen oder mehrere kieselsäurebasierende Füllstoffe. Vorzugsweise werden dafür folgende Stoffe verwendet:
- Kieselsäure, insbesondere Fällungskieselsäure oder pyrogene Kieselsäure, hergestellt z.B. durch Fällung von Lösungen von Silikaten oder Flammenhydrolyse von Siliziumhalogeniden mit spezifischen Oberflächen von 5 - 1000, vorzugsweise 20 - 400 m²/g (BET-Oberfläche) und mit Primärteilchengrößen von 10 - 400 nm. Die Kieselsäuren können gegebenenfalls auch als Mischoxide mit anderen Metalloxiden wie Al-, Mg-, Ca-, Ba-, Zn-, Zr-, Ti-Oxiden vorliegen.
- synthetische Silikate, wie Aluminiumsilikat, Erdalkalisilikate wie Magnesium- oder Calciumsilikat, mit BET-Oberflächen von 20 - 400 m²/g und Primärteilchengröße von 10 - 400 nm,
- natürliche Silikate, wie Kaolin und andere natürliche vorkommende Kieselsäuren,
- Glasfasern auch in Formen von Matten und Strängen,
- Mikroglaskugeln.

Es können selbstverständlich zusätzliche Füllstoffe verwendet werden. Insbesondere sind hierfür nach dem Flammruß, Furnace- oder Gasruß-Verfahren hergestellt Ruße geeignet, welche BET-Oberflächen von 20 - 200 m²/g besitzen, wie SAF-, ISAF-, IISAF-, HAF-, FEF- oder GPF-Ruße.

Der Gesamtgehalt an Füllstoffen beträgt vorzugsweise 10 bis 200 phr, besonders bevorzugt 50 bis 160 phr und ganz besonders bevorzugt 60 bis 120 phr.

Eine besonders bevorzugte Ausführungsform stellt die Kombination von Kieselsäure, Ruß und erfindungsgemäßer Polysulfidmischung dar. Dabei kann das Verhältnis von Kieselsäure zu Ruß in beliebigen Grenzen variieren, wobei für die Anwendung in Reifen ein Kieselsäure : Ruß - Gewichtsverhältnis von 20 : 1 bis 1,5 : 1 bevorzugt ist.

In einer bevorzugten Ausführungsform enthalten die erfindungsgemäßen Kautschukmischungen auch einen oder mehrere Vernetzer. Hierfür sind insbesondere Schwefel-basierende oder peroxidische Vernetzer geeignet, wobei Schwefel-basierte Vernetzer besonders bevorzugt sind.

Als peroxidische Vernetzer werden vorzugsweise Bis(2,4-dichlorbenzyl)peroxid, Dibenzoylperoxid, Bis(4-chlorbenzoyl)peroxid, 1,1-Bis(t-butylperoxy)-3,3,5-trimethyl-cylohexan, tert-Butylperbenzoat, 2,2-Bis(t-butylperoxy)butan, 4,4-Di-tert-butylperoxynonylvalerat, Dicumylperoxid, 2,5-Dimethyl-2,5-di(tert-butylperoxy)hexan, tert-Butylcumylperoxid, 1,3-Bis(t-butylperoxyisopropyl)benzol, Di-tert-butylperoxid und 2,5-Dimethyl-2,5-di(tert-butylperoxy)-3-hexin eingesetzt.

Es kann vorteilhaft sein, neben diesen peroxidischen Vernetzern noch weitere Zusätze zu verwenden, mit deren Hilfe die Vernetzungsausbeute erhöht werden kann: Hierfür sind beispielsweise Triallylisocyanurat, Triallylcyanurat, Trimethylolpropantri(meth)acrylat, Triallyltrimellitat, Ethylenglycoldi(meth)acrylat, Butandioldi(meth)acrylat, Trimetylolpropan-tri(meth)acrylat, Zinkdiacrylat, Zinkdimethacrylat, 1,2-Polybutadien oder N,N'-m-Phenylen-dimaleinimid geeignet.

Als Vernetzer kann Schwefel in elementarer löslicher oder unlöslicher Form oder in Form von Schwefelspendern eingesetzt werden. Als Schwefelspender kommen beispielsweise Dimorpholyldisulfid (DTDM), 2-Morpholinodithiobenzothiazol (MBSS), Caprolactamdisulfid, Dipentamethylenthiuramtetrasulfid (DPTT) und Tetramethylthiuramdisulfid (TMTD) in Frage.

Grundsätzlich kann die Vernetzung der erfindungsgemäßen Kautschukmischungen mit Schwefel oder Schwefelspendern allein erfolgen, oder zusammen mit Vulkanisationsbeschleunigern, wofür z.B. Dithiocarbamate, Thiurame, Thiazole, Sulfenamide, Xanthogenate, bi- oder polycyclische Amine, Guanidinderivate, Dithiophosphate, Caprolactame und Thioharnstoffderivate geeignet sind. Weiterhin sind auch Zinkdiamindiisocyanat, Hexamethylentetramin, 1,3-Bis(citraconimidomethyl)benzol sowie zyklische Disulfane geeignet. Bevorzugt enthalten die erfindungsgemäßen Kautschukmischungen Schwefel-basierende Vernetzer und Vulkanisationsbeschleuniger.

Besonders bevorzugt werden als Vernetzungsmittel Schwefel, Magnesiumoxid und/oder Zinkoxid eingesetzt, denen die bekannten Vulkanisationsbeschleuniger, wie Merkaptobenzothiazole, Thiazolsulfenamide, Thiurame, Thiocarbamate, Guanidine, Xanthogenate und Thiophosphate zugesetzt werden.

Die Vernetzungsmittel und Vulkanisationsbeschleuniger werden bevorzugt in Mengen von 0,1 bis 10 phr, besonders bevorzugt von 0,1 bis 5 phr, eingesetzt.

Die erfindungsgemäßen Kautschukmischungen können weitere Kautschukhilfsmittel enthalten, wie Reaktionsbeschleuniger, Alterungsschutzmittel, Wärmestabilisatoren, Lichtschutzmittel, Antioxidantien, insbesondere Ozonschutzmittel, Flammschutzmittel, Verarbeitungshilfsmittel, Schlagzähfestigkeitsverbesserer, Weichmacher, Tackifier, Treibmittel, Farbstoffe, Pigmente, Wachse, Streckmittel, organische Säuren, Verzögerer, Metalloxide und Aktivatoren, insbesondere Triethanolamin, Polyethylenglykol, Hexantriol und Reversionsschutzmittel.

Diese Kautschukhilfsmittel werden in üblichen Mengen eingesetzt, die sich u. a. nach dem Verwendungszweck der Vulkanisate richten. Übliche Mengen sind 0,1 bis 30 phr.

Als Alterungsschutzmittel werden bevorzugt alkylierte Phenole, styrolisiertes Phenol, sterisch gehinderte Phenole wie 2,6-Di-tert.-butylphenol, 2,6-Di-tert-butyl-p-kresol (BHT), 2,6-Di-tert.-butyl-4-ethylphenol, estergruppenhaltige sterisch gehinderte Phenole, thioetherhaltige sterisch gehinderte Phenole, 2,2'-Methylen-bis-(4-methyl-6-tert-butylphenol) (BPH) sowie sterisch gehinderte Thiobisphenole verwendet.

Falls eine Verfärbung des Kautschuks ohne Bedeutung ist, können auch aminische Alterungsschutzmittel z.B. Mischungen aus Diaryl-p-phenylendiaminen (DTPD), octyliertes Diphenylamin (ODPA), Phenyl-α-naphthylamin (PAN), Phenyl-β-naphthylamin (PBN), vorzugsweise solche auf Phenylendiaminbasis, z. B. N-Isopropyl-N'-phenyl-p-phenylendiamin, N-1,3-Dimethylbutyl-N'-Phenyl-p-phenylendiamin (6PPD), N-1,4-Dimethylpentyl-N'-phenyl-p-phenylendiamin (7PPD), N,N'-bis-(1,4-Dimethylpentyl)-p-phenylendiamin (77PD) eingesetzt werden.

Weitere Alterungsschutzmittel sind Phosphite wie Tris-(nonylphenyl)phosphit, polymerisiertes 2,2,4-Trimethyl-1,2-dihydrochinolin (TMQ), 2-Mercaptobenzimidazol (MBI), Methyl-2-Mercaptobenzimidazol (MMBI), Zinkmethylmercaptobenzimidazol (ZMMBI), welche meist in Kombination mit obigen phenolischen Alterungsschutzmitteln eingesetzt werden. TMQ, MBI und MMBI werden vor allem für NBR-Kautschuke verwendet, welche peroxidisch vulkanisiert werden.

Die Ozonbeständigkeit kann durch Antioxidantien wie beispielsweise N-1,3-Dimethylbutyl-N'-phenyl-p-phenylendiamin (6PPD), N-1,4-Dimethylpentyl-N'-phenyl-p-phenylendiamin (7PPD), N,N'-Bis- (1,4-dimethylpentyl)-p-phenylendiamin (77PD), Enolether oder cyclische Acetale. verbessert werden.

Verarbeitungshilfsmittel sollen zwischen den Kautschuk-Partikeln wirksam werden und Reibungskräften beim Mischen, Plastifizieren und Verformen entgegenwirken. Als Verarbeitungshilfsmittel können die erfindungsgemäßen Kautschukmischungen alle für die Verarbeitung von Kunststoffen üblichen Gleitmittel enthalten, wie beispielsweise Kohlenwasserstoffe, wie Öle, Paraffine und PE-Wachse, Fettalkohole mit 6 bis 20 C-Atomen, Ketone, Carbonsäuren, wie Fettsäuren und Montansäuren, oxidiertes PE-Wachs, Metallsalze von Carbonsäuren, Carbonsäureamide sowie Carbonsäureester, beispielsweise mit den Alkoholen Ethanol, Fettalkoholen, Glycerin, Ethandiol, Pentaerythrit und langkettigen Carbonsäuren als Säurekomponente.

Um die Entflammbarkeit zu vermindern und die Rauchentwicklung beim Verbrennen zu verringern, kann die erfindungsgemäße Kautschukmischungszusammensetzung auch Flammschutzmittel enthalten. Hierfür werden beispielsweise Antimontrioxid, Phosphorsäureester, Chlorparaffin, Aluminiumhydroxid, Borverbindungen, Zinkverbindungen, Molybdäntrioxid, Ferrocen, Calciumcarbonat oder Magnesiumcarbonat verwendet.

Vor der Vernetzung können dem Kautschukvulkanisat auch weitere Kunststoffe beigefügt werden, welche beispielsweise als polymere Verarbeitungshilfsmittel oder Schlagzähigkeitsverbesserer wirken. Diese Kunststoffe werden bevorzugt gewählt aus der Gruppe bestehend aus den Homo- und Copolymeren auf Basis von Ethylen, Propylen, Butadien, Styrol, Vinylacetat, Vinylchlorid, Glycidylacrylat, Glycidylmethacrylat, Acrylaten und Methacrylaten mit Alkoholkomponenten von verzweigten oder unverzweigten C₁- bis C₁₀-Alkoholen, wobei Polyacrylate mit gleichen oder verschiedenen Alkoholresten aus der Gruppe der C₄- bis C₈-Alkohole, insbesondere des Butanols, Hexanols, Octanols und 2-Ethylhexanols, Polymethylmethacrylat, Methylmethacrylat-Butylacrylat-Copolymere, Methylmethacrylat-Butylmethacrylat-Copolymere, Ethylen-Vinylacetat-Copolymere, chloriertes Polyethylen, Ethylen-Propylen-Copolymere, Ethylen-Propylen-Dien-Copolymere besonders bevorzugt sind.

In einer bevorzugten Ausführungsform enthält die erfindungsgemäße Kautschukmischung 0,1 bis 15 phr des Reversionsschutzmittels 1,6-Bis(N,N-dibenzylthiocarbamoyldithio)-hexan (CAS-Nr.: 151900-44-6), wodurch eine weitere Verringerung des Rollwiderstands ermöglicht wird.

Bevorzugt zeichnen sich die erfindungsgemäßen Kautschukmischungen dadurch aus, dass ein daraus bei 170°C/t95 geheiztes Vulkanisat einen Verlustfaktor tan δ bei 60°C von < 0,12 und eine Shore A Härte bei 23°C von > 68 aufweist. In Kombination damit können durch die erfindungsgemäßen Kautschukmischungen auch eine Ausvulkanisationszeit von kleiner 2000 Sekunden und besonders bevorzugt eine Anvulkanisationszeit zwischen 500 - 1000 Sekunden erreicht werden.

Ein weiterer Gegenstand der vorliegenden Erfindung ist ein Verfahren zur Herstellung von Kautschukmischungen, durch Mischen von mindestens einem Kautschuk mit einer erfindungsgemäßen Polysulfidmischung. Vorzugsweise werden zudem mindestens ein kieselsäurebasierender Füllstoff, sowie mindestens ein schwefelhaltiges Alkoxysilan beigemischt. Bevorzugt werden dabei 10 bis 150 phr, besonders bevorzugt 30 bis 120 phr und ganz besonders bevorzugt 50 bis 100 phr Füllstoff, 0,1 bis 15 phr, besonders bevorzugt 0,3 bis 7 phr, ganz besonders bevorzugt 0,5 bis 3 phr und am meisten bevorzugt 0,7 bis 1,5 phr erfindungsgemäße Polysulfidmischung sowie 2 bis 20 phr, besonders bevorzugt 3-11 phr und ganz besonders bevorzugt 5 bis 8 phr des schwefelhaltigen Alkoxysilans eingesetzt. Weiterhin können im Mischverfahren die oben genannten zusätzlichen Füllstoffe, Vernetzer, Vulkanisationsbeschleuniger und Kautschukhilfsmittel, vorzugsweise in den oben angegebenen Mengen zugesetzt werden.

Bei dem mehrstufigen Mischverfahren erfolgt die Zugabe der erfindungsgemäßen Polysulfidmischung bevorzugt im ersten Teil des Mischprozesses und die Zugabe von einem oder mehreren Vernetzern, insbesondere Schwefel, und ggf. Vulkanisationsbeschleuniger in einer späteren Mischstufe. Die Temperatur der Kautschukmasse beträgt dabei vorzugsweise 100 bis 200 °C, besonders bevorzugt 120°C bis 170°C. Die Scherraten bei der Mischung betragen 1 bis 1000 sec⁻¹, bevorzugt 1 bis 100 sec⁻¹. In einer bevorzugten Ausführungsform wird die Kautschukmischung nach der ersten Mischstufe abgekühlt und der Vernetzer und ggf. Vernetzungsbeschleuniger und/oder Zusätze mit deren Hilfe die Vernetzungsausbeute erhöht wird, in einer späteren Mischstufe bei < 140°C, vorzugsweise < 100°C zugegeben. Die Zugabe der erfindungsgemäßen Polysulfidmischung in einer späteren Mischstufe und bei tieferen Temperaturen wie 40 bis 100°C ist ebenfalls möglich z.B. zusammen mit Schwefel und Vernetzungsbeschleuniger.

Die Abmischungen des Kautschuks mit dem Füllstoff und der erfindungsgemäßen Polysulfidmischung kann in üblichen Mischaggregaten, wie Walzen, Innenmischer und Mischextruder, durchgeführt werden.

Die optionale Zugabe von 1,6-Bis(N,N-dibenzylthiocarbamoyldithio)hexan findet vorzugsweise in der ersten Stufe des mehrstufigen Mischverfahrens statt.

Ein weiterer Gegenstand der vorliegenden Erfindung ist ein Verfahren zur Vulkanisation der erfindungsgemäßen Kautschukmischungen, welche bevorzugt bei Massetemperaturen von 100 bis 200 °C, besonders bevorzugt bei 130 bis 180 °C durchgeführt wird. In einer bevorzugten Ausführungsform geschieht die Vulkanisation bei einem Druck von 10 bis 200 bar.

Die vorliegende Erfindung umfasst auch Kautschukvulkanisate erhältlich durch Vulkanisation der erfindungsgemäßen Kautschukmischungen. Weiterhin umfasst die vorliegende Erfindung Kautschukprodukte enthaltend diese Vulkanisate, insbesondere Reifen, da entsprechende Reifen den Vorteil einer hohen Härte gepaart mit einem guten Rollwiderstand aufweisen.

Die Ausrüstung von Fahrzeugen insbesondere Kraftfahrzeugen mit Reifen, welche die erfindungsgemäßen Vulkanisate enthalten, führt zu einem geringeren Energieaufwand beim Betrieb dieser Fahrzeuge, wodurch bei Kraftfahrzeugen mit Verbrennungsmotoren ein geringerer Kraftstoffverbrauch, bei Fahrzeugen mit Elektroantrieb eine größere Reichweite und bei muskelbetriebenen Fahrzeugen eine geringere Anstrengung und/oder eine höhere Geschwindigkeit ermöglicht wird. Daher umfasst die vorliegende Erfindung auch Fahrzeuge enthaltend Kautschukprodukte, welche die erfindungsgemäßen Vulkanisate beinhalten.

Die erfindungsgemäßen Kautschukvulkanisate eignen sich zur Herstellung von Formkörpern mit verbesserten Eigenschaften, z.B. für die Herstellung von Kabelmäntel, Schläuchen, Treibriemen, Förderbändern, Walzenbelägen, Reifen, Schuhsohlen, Dichtungsringen und Dämpfungselementen.

Das erfindungsgemäße Kautschukvulkanisat kann zudem zur Herstellung von Schaumstoffen verwendet werden. Dazu werden ihr chemisch oder physikalisch wirkende Treibmittel zugefügt. Als chemisch wirkende Treibmittel kommen alle für diesen Zweck bekannten Substanzen in Betracht, wie beispielsweise Azodicarbonamid, p-Toluolsulfonylhydrazid, 4,4'-Oxybis(benzolsulfohydrazid), p-Toluolsulfonylsemicarbazid, 5-Phenyltetrazol, N,N'-Dinitroso-pentamethylentetramin, Zinkcarbonat oder Natriumhydrogencarbonat sowie diese Substanzen enthaltende Mischungen. Als physikalisch wirkende Treibmittel sind beispielsweise Kohlendioxid oder Halogenkohlenwasserstoffe geeignet.

Ein weiterer Gegenstand der vorliegenden Erfindung ist die Verwendung der erfindungsgemäßen Polysulfidmischung zur Herstellung von Kautschukmischungen, und deren Vulkanisaten insbesondere zur Herstellung von Kautschukmischungen enthaltend zumindest jeweils einen Kautschuk, ein schwefelhaltiges Alkoxysilan und einen kieselsäurebasierenden Füllstoff.

Darüber hinaus betrifft die vorliegende Erfindung die Verwendung der erfindungsgemäßen Polysulfidmischung zur Herstellung von Additivzusammensetzungen für Kautschuke.

Überraschenderweise wurde gefunden, dass Additivzusammensetzungen für Kautschuke enthaltend zumindest ein schwelfelhaltiges Alkoxysilan, insbesondere Bis-(triethoxysilylpropyl)tetrasulfan, Bis-(triethoxysilylpropyl)disulfan, 3-(Triethoxysilyl)-1-propanthiol, polyetherfunktionalisiertes Mercaptosilan oder thioesterfunktionalisiertes Alkoxysilan und die erfindungsgemäße Polysulfidmischung trotz der reaktiven Gruppen eine ausreichende Verträglichkeit der Komponenten aufweisen, wodurch eine homogene Einbringung in Kautschukmischungen und eine exakte Dosierung im gewünschten Verhältnis ermöglicht wird. Daher umfasst die vorliegende Erfindung auch solche Additivzusammensetzungen für Kautschuke, sowie die Verwendung der erfindungsgemäßen Polysulfidmischungen zur Herstellung solcher Additivzusammensetzungen. In diesen Additivzusammensetzungen beträgt das Gewichtsverhältnis von Alkoxysilan, insbesondere von Bis-(triethoxysilylpropyl)tetrasulfan und/oder von Bis-(triethoxysilylpropyl)disulfan zu den erfindungsgemäßen Polysulfidmischungen vorzugsweise 1,5:1 bis 20:1, besonders bevorzugt 3:1 bis 15:1 und ganz besonders bevorzugt 5:1 bis 10:1.

Zudem umfasst die vorliegende Erfindung ein Verfahren zur Herstellung von Additivzusammensetzungen für Kautschuke, dadurch gekennzeichnet, dass man schwelfelhaltige Alkoxysilane mit den erfindungsgemäßen Polysulfidmischungen mischt.

Weiterhin umfasst die vorliegende Erfindung auch ein Verfahren zur Verringerung des Rollwiderstands von Reifen, bei welchem einer nicht oder teilvernetzten Kautschukmischung welche als Ausgangsmaterial für zumindest Teile des Reifen dient, eine erfindungsgemäße Polysulfidmischung beimengt und die Mischung anschließend vulkanisiert.

### Bestimmung der Eigenschaften von Kautschukmischung bzw. Vulkanisaten:

### Mooney-Viskositätsmessung:

Die Viskosität lässt sich aus der Kraft, die Kautschuke (und Kautschukmischungen) ihrer Verarbeitung entgegensetzen, direkt bestimmen. Beim Scherscheibenviskosimeter nach Mooney wird eine geriffelte Scheibe oben und unten mit Probensubstanz umschlossen und in einer beheizbaren Kammer mit etwa zwei Umdrehungen in der Minute bewegt. Die hierzu erforderliche Kraft wird als Drehmoment gemessen und entspricht der jeweiligen Viskosität. Die Probe wird in der Regel eine Minute lang auf 100°C vorgewärmt; die Messung dauert weitere 4 Minuten, wobei die Temperatur konstant gehalten wird. Die Viskosität wird zusammen mit den jeweiligen Prüfbedingungen angegeben, beispielsweise ML (1+4) 100°C (Mooney viscosity, large rotor, Vorwärmzeit und Prüfzeit in Minuten, Prüftemperatur).

### Scorch-Verhalten (Anvulkanisationszeit t 5):

Des Weiteren kann mit der gleichen Prüfung wie oben beschrieben auch das Scorch-Verhalten einer Mischung gemessen werden. Die gewählte Temperatur betrug 130°C. Der Rotor läuft solang, bis der Drehmomentswert nach Durchlaufen eines Minimums auf 5 Mooney-Einheiten relativ zum Minimumswert angestiegen ist (t5). Je größer der Wert ist (Einheit Sekunden), umso langsamer findet die Anvulkanisation statt. In der Praxis ist meist eine Anvulkanisationszeit von mehr als 300 Sekunden vorteilhaft, welche unter Berücksichtigung von Verarbeitungssicherheit und Zeitaufwand aber weniger als 1000 Sekunden betragen sollte.

### Rheometer (Vulkameter) Ausvulkanisationszeit 170°C/t95:

Der Vulkanisationsverlauf am MDR (moving die rheometer) und dessen analytischen Daten werden an einem Monsanto-Rheometer MDR 2000 nach ASTM D5289-95 gemessen. Als Ausvulkanisationszeit, wird die Zeit bestimmt, bei der 95% des Kautschuks vernetzt ist. Die gewählte Temperatur betrug 170°C.

### Bestimmung der Härte:

Zur Bestimmung der Härte der erfindungsgemäßen Kautschukmischung wurden 6 mm starke Walzfelle aus der Kautschukmischung gemäß Rezepturen der Tabelle 1 hergestellt. Aus den Walzfellen wurden Prüfkörper mit 35 mm Durchmesser geschnitten, deren Shore-Härte A mittels eines digitalen Shore-Härte-Testers (Zwick GmbH & Co. KG, Ulm) bestimmt wurden. Die Härte eines Kautschukvulkanisates gibt einen ersten Hinweis über dessen Steifigkeit wieder.

### Zugversuch:

Der Zugversuch dient direkt zur Ermittlung der Belastungsgrenzen eines Elastomers und erfolgt nach DIN 53504. Die Längenausdehnung beim Bruch wird auf die Ausgangslänge bezogen und entspricht der Bruchdehnung. Weiterhin wird auch die Kraft beim Erreichen bestimmter Dehnungsstufen, meist 50, 100, 200 und 300% bestimmt und als Spannungswert ausgedrückt (Zugfestigkeit bei der angegebenen Dehnung von 300% oder Modul 300).

### Dyn. Dämpfung:

Dynamische Prüfverfahren werden zur Charakterisierung des Verformungsverhaltens von Elastomeren unter periodisch veränderten Belastungen verwendet. Eine von außen angebrachte Spannung verändert die Konformation der Polymerkette. Der Verlustfaktor tan δ wird dabei indirekt über das Verhältnis zwischen Verlustmodul G" und Speichermodul G' bestimmt. Der Verlustfaktor tan δ bei einer Temperatur im Bereich von 60°C geht mit dem Rollwiderstand einher und sollte möglichst niedrig sein.

### Abrieb:

Der Abrieb gibt einen Hinweis auf die Abnutzung und damit auf die Lebensdauer eines Produktes. Der Abrieb wurde nach DIN 53516 bestimmt. Aus ökonomischen und ökologischen Gründen ist ein niedriger Wert anzustreben.

### Beispiel 1

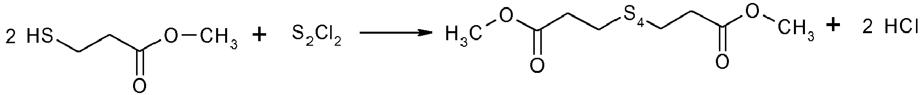

| | |
|---|---|
| | Apparatur:500ml Vierhalskolben mit Thermometer, Tropftrichter mit Druckausgleich, Rückflusskühler mit Gasableitungsansatz (Blasenzähler) und Schlauch, Rührwerk, Gaseinleitungsrohr |
| Vorlage: | 92 g (0,75 mol) 3-Mercaptopropionsäuremethylester (Acros, ≥ 98%) 250 ml Cyclohexan (p.A., Fa. Merck über Molekularsieb getrocknet) |
| Zulauf: | 51,2 g (0,375 mol) Dischwefeldichlorid (≥ 99%, Fa. Merck) |

In der stickstoffgespülten Apparatur wurde getrocknetes Cyclohexan und 3-Mercaptopropion-säuremethylester vorgelegt. Nach vollständiger Lösung des 3-Mercaptopropionsäuremethylesters wurde das Dischwefeldichlorid unter Stickstoffdurchleitung bei einer Temperatur von 5-10°C innerhalb von ca. einer 1h zugetropft. Die Dosiergeschwindigkeit wurde so eingestellt, dass eine Temperatur von 10°C nicht überschritten wurde. Nach beendeter Reaktion wurde unter Stickstoffdurchleitung bei Raumtemperatur über Nacht nachgerührt. Anschließend wurde die Reaktionslösung am Rotationsverdampfer bei 50°C eingeengt und im Vakuumtrockenschrank bei 60°C bis zur Gewichtskonstanz nachgetrocknet.

| | |
|---|---|
| Ausbeute: | 108,4 g (95,6%) einer Polysulfidmischung der idealisierten Formel 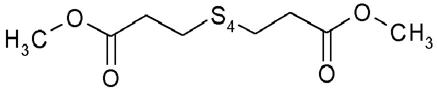 |

| | | | |
|---|---|---|---|
| Elementaranalyse: | | | |
| C: 31,9% | H: 5,3% | O: 21,7% | S: 42,0% |

Das Produkt wurde durch RP-HPLC und Time-of-Flight Mass Spectrometry (TOF MS) analysiert.

Die Prozentangaben zu den Verbindungen der Formel (I) ergeben sich aus den Flächenprozenten der HPLC-Messung mit RI-Detektor.

### < 1% Verbindung (II), 16% Verbindung (III), 65% Verbindung (IV), 15% Verbindung (V), 4% Verbindung (VI)

### Beispiel 2

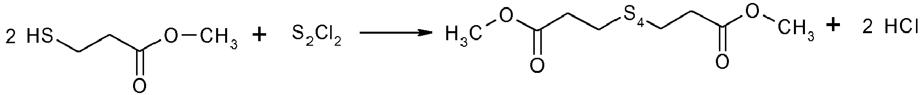

| | |
|---|---|
| Apparatur: | 500ml Vierhalskolben mit Thermometer, Tropftrichter mit Druckausgleich, Rückflusskühler mit Gasableitungsansatz (Blasenzähler) und Schlauch, Rührwerk, Gaseinleitungsrohr |
| Vorlage: | 92 g (0,75 mol) 3-Mercaptopropionsäuremethylester (Acros, ≥ 98%) 250 ml Cyclohexan (p.A., Fa. Merck über Molekularsieb getrocknet) |
| Zulauf: | 51,2 g (0,375 mol) Dischwefeldichlorid (≥ 99%, Fa. Merck) |

In der stickstoffgespülten Apparatur wurden getrocknetes Cyclohexan und 3-Mercaptopropion-säuremethylester vorgelegt. Nach vollständiger Lösung des 3-Mercaptopropionsäuremethylesters wurde das Dischwefeldichlorid unter Stickstoffdurchleitung bei einer Temperatur von 20-25°C innerhalb von ca. 30min zugetropft. Die Dosiergeschwindigkeit wurde so eingestellt, dass eine Temperatur von 25°C nicht überschritten wurde. Nach beendeter Reaktion wurde unter Stickstoffdurchleitung bei Raumtemperatur über Nacht nachgerührt. Anschließend wurde die Reaktionslösung am Rotationsverdampfer bei ca. 30°C eingeengt.

| | |
|---|---|
| Ausbeute: | 115g (101,4%) einer Polysulfidmischung der idealisierten Formel 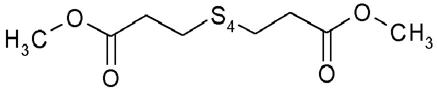 |

| | | | | |
|---|---|---|---|---|
| Elementaranalyse: | | | | |
| C: 31,9% | H: 4,5% | O: 21,7% | S: 42,1% | CI: < 10ppm |

Das Produkt wurde durch RP-HPLC und Time-of-Flight Mass Spectrometry (TOF MS) analysiert.

Die Prozentangaben zu den Verbindungen der Formel (I) ergeben sich aus den Flächenprozenten der HPLC-Messung mit RI-Detektor.

### < 1% Verbindung (II), 1% Verbindung (III), 96% Verbindung (IV), 2% Verbindung (V), < 1% Verbindung (VI)

### Beispiel 3

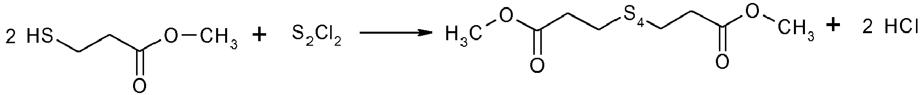

| | |
|---|---|
| Apparatur: | 500ml Vierhalskolben mit Thermometer, Tropftrichter mit Druckausgleich, Rückflusskühler mit Gasableitungsansatz (Blasenzähler) und Schlauch, Rührwerk, Gaseinleitungsrohr |
| Vorlage: | 92 g (0,75 mol) 3-Mercaptopropionsäuremethylester (Acros, ≥ 98%) 200 g Polysulfidmischung hergestellt analog Beispiel 2 |
| Zulauf: | 51,2 g (0,375 mol) Dischwefeldichlorid (≥ 99%, Fa. Merck) |

In der stickstoffgespülten Apparatur wurde die Polysulfidmischung (hergestellt analog Beispiel 2) und 3-Mercaptopropionsäuremethylester vorgelegt. Nach vollständiger Lösung des 3-Mercaptopropionsäuremethylesters wurde das Dischwefeldichlorid unter Stickstoffdurchleitung bei einer Temperatur von 20-25°C innerhalb von ca. 30min zugetropft. Die Dosiergeschwindigkeit wurde so eingestellt, dass eine Temperatur von 25°C nicht überschritten wurde. Nach beendeter Reaktion wurde unter Stickstoffdurchleitung bei Raumtemperatur über Nacht nachgerührt. Anschließend wurde die Reaktionslösung am Rotationsverdampfer 2h bei ca. 30°C nachbehandelt.

| | |
|---|---|
| Ausbeute: | 200 g + 115 g (101,4%) einer Polysulfidmischung der idealisierten Formel 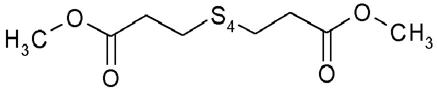 |

| | | | | |
|---|---|---|---|---|
| Elementaranalyse: | | | | |
| C: 32,0% | H: 4,8% | O: 21,8% | S: 41,9% | CI: < 10ppm |

Das Produkt wurde durch RP-HPLC analysiert. Die Prozentangaben zu den Verbindungen der Formel (I) ergeben sich aus den Flächenprozenten der HPLC-Messung mit RI-Detektor.

### < 1% Verbindung (II), 1% Verbindung (III), 96% Verbindung (IV), 2% Verbindung (V), < 1% Verbindung (VI)

HPLC-System mit Vakuum-Degaser, Pumpe, Säulenofen, Injektionssystem und RI-Detektor.

| | |
|---|---|
| Säulentyp: | Eclipse XDB-C8, 5µm |
| Säulenlänge: | 150 mm |
| Säuleninnendurchmesser: | 4,6 mm |
| Mobile Phase: | 33% Wasser, 67% Methanol |
| Säulentemperatur: | 35°C |
| Fließgeschwindigkeit: | 0,8 ml/min |
| Laufzeit: | 30 min |
| Injektionsvolumen: | 2 µl |
| RI-Detektor: | Agilent 1100 Series G1362A |

10ml Tetrahydrofuran wurden in einer 25ml Rollrandflasche vorgelegt und ca. 300 µl der Probe zugegeben, homogenisiert und die Lösung direkt im Anschluss chromatographiert. Die Auswertung erfolgt in Flächenprozent.

### Herstellung von Kautschukmischungen und Kautschukvulkanisaten

Die in Tabelle 1 aufgeführten Kautschuk-Rezepturen wurden jeweils gemäß nachfolgend beschriebenen, mehrstufigen Verfahren gemischt.

### 1. Mischstufe:

■ BUNA^{®} CB 24 und BUNA^{®} VSL 5025-2 wurde in einem Innenmischer vorgelegt und ca. 30 Sekunden gemischt
■ Zugabe zwei Drittel VULKASIL^{®} S, zwei Drittel SI^{®} 69, zwei Drittel der Gesamtmenge an erfindungsgemäßer Polysulfidmischung, mischen für ca. 60 Sekunden
■ Zugabe ein Drittel VULKASIL^{®} S, ein Drittel SI^{®} 69, ein Drittel der Gesamtmenge an erfindungsgemäßer Polysulfidmischung, sowie TUDALEN 1849-1, mischen ca. 60 Sekunden

Zugabe von CORAX^{®} N 339, EDENOR^{®} C 18 98-100, VULKANOX^{®} 4020/LG, VULKANOX^{®} HS/LG, ZINKWEISS ROTSIEGEL sowie ANTILUX^{®} 654, mischen für ca. 60 Sekunden. Die Mischung erfolgte bei einer Temperatur von 150°C.

### 2. Mischstufe:

Nach Abschluss der ersten Mischstufe wurde das Mischstück von einem nachgeschalteten Walzwerk aufgenommen und zu einer Platte ausgeformt und für 24 Stunden bei Raumtemperatur gelagert. Die Verarbeitungstemperaturen lagen hierbei unter 60°C.

### 3. Mischstufe:

In der dritten Mischstufe erfolgte ein Nachzwicken bei 150°C in einem Kneter.

### 4. Mischstufe:

Zugabe der Zusatzstoffe MAHLSCHWEFEL 90/95 CHANCEL, VULKACIT^{®} CZ/C, VULKACIT^{®} D/C auf einer Walze bei Temperaturen unter 80°C.

Die Kautschukmischungen wurden anschließend bei 170°C ausvulkanisiert. Die Eigenschaften der hergestellten Kautschukzubereitungen und deren Vulkanisate sind in Tabelle 2 angegeben.

**Tabelle 1: Kautschuk-Rezeptur**

| | **Referenz** | **Kautschuk-rezeptur 1** | **Kautschuk-rezeptur 2** | **Kautschuk-rezeptur 3** |
|---|---|---|---|---|
| BUNA CB 24 | 30 | 30 | 30 | 30 |
| BUNA VSL 5025-2 | 96 | 96 | 96 | 96 |
| CORAX N 339 | 6,4 | 6,4 | 6,4 | 6,4 |
| VULKASIL S | 80 | 80 | 80 | 80 |
| TUDALEN 1849-1 | 8 | 8 | 8 | 8 |
| EDENOR C 18 98-100 | 1 | 1 | 1 | 1 |
| VULKANOX 4020/LG | 1 | 1 | 1 | 1 |
| VULKANOX HS/LG | 1 | 1 | 1 | 1 |
| ZINKWEISS ROTSIEGEL | 2,5 | 2,5 | 2,5 | 2,5 |
| ANTILUX 654 | 1,5 | 1,5 | 1,5 | 1,5 |
| SI 69 | 6,4 | 6,4 | 6,4 | 6,4 |
| VULKACIT D/C | 2 | 2 | 2 | 2 |
| VULKACIT CZ/C | 1,5 | 1,5 | 1,5 | 1,5 |
| MAHLSCHWEFEL 90/95 CHANCEL | 1,5 | 1,5 | 1,5 | 1,5 |
| Verbindung 1 | | 1 | | |
| Verbindung 2 | | | 1 | |
| Verbindung 3 | | | | 1 |

**Mengenangaben in phr (Gewichtsteile pro 100 Teile Kautschuk)**

| **Handelsname** | **Erläuterung** | **Hersteller/Vertrieb** |
|---|---|---|
| BUNA CB 24 | BR | Lanxess Deutschland GmbH |
| BUNA VSL 5025-2 | SBR | Lanxess Deutschland GmbH |
| CORAX N 339 | Ruß | Degussa-Evonik GmbH |
| VULKASIL S | Kieselsäure | Lanxess Deutschland GmbH |
| TUDALEN 1849-1 | Mineralöl | Hansen&Rosenthal KG |
| EDENOR C 18 98-100 | Stearinsäure | Cognis Deutschland GmbH |
| VULKANOX 4020/LG | N-1,3-Dimethylbutyl-N-phenyl-p-phenylendiamin | Lanxess Deutschland GmbH |
| VULKANOX HS/LG | 2,2,4-Trimethyl-1,2-dihydrochinolin polymerisiert | Lanxess Deutschland GmbH |
| ZINKWEISS ROTSIEGEL | Zinkoxid | Grillo Zinkoxid GmbH |
| ANTILUX 654 | Lichtschutzwachs | RheinChemie Rheinau GmbH |
| SI 69 | Bis(triethoxysilylpropyl)tetrasulfi d | Evonik Industries |
| VULKACIT D/C | 1,3-Diphenylguanidin | Lanxess Deutschland GmbH |
| VULKACIT CZ/C | N-cyclohexyl-2-benzothiazol-sulfenamid | Lanxess Deutschland GmbH |
| MAHLSCHWEFEL 90/95 CHANCEL | Schwefel | Solvay Deutschland GmbH |

**Tabelle 2: Zusammenstellung der Ergebnisse**

| **Parameter** | **Einheit** | **DIN** | **Referenz** | **Kautschuk -rezeptur 1** | **Kautschuk -rezeptur 2** |
|---|---|---|---|---|---|
| Mooney Viskosität (ML 1+4) | [ME] | 53523 | 95 | 82 | 87 |
| Mooney Anvulkanisation bei 130°C (t5) | sec | ASTM D 5289-95 | 1253 | 1244 | 884 |
| Ausvulkanisation bei 170°C /t95 | sec | 53529 | 1417 | 1617 | 1648 |
| Shore A Härte bei 23°C | [Shore A] | 53505 | 66 | 72 | 69 |
| Modul 300 | MPa | 53504 | 15 | 17 | 14 |
| Bruchdehnung | % | 53504 | 349 | 346 | 350 |
| Zugfestigkeit | MPa | 53504 | 19 | 20 | 17 |
| Abrieb | mm³ | 53516 | 74 | 95 | 73 |
| Rollwiderstand (tan δ (60 °C)) | - | | 0,133 | 0,168 | 0,107 |

## Patentansprüche

1. Polysulfidmischung enthaltend zwei oder mehr Verbindungen der Formel (I) 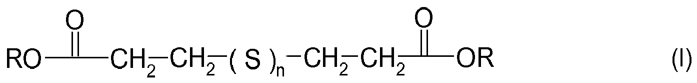 wobei
jeder der beiden Reste R identisch oder verschieden, vorzugsweise identisch ist und für Methyl, Ethyl, n-Propyl, 2-Propyl, n-Butyl, Isobutyl, t-Butyl oder C₅-Alkyl, C₆-Alkyl, C₇-Alkyl oder C₈-Alkyl vorzugsweise Methyl, Ethyl, n-Propyl, n-Butyl oder Isooctyl, besonders bevorzugt Methyl steht und
n = 2 bis 6,
**dadurch gekennzeichnet, dass** der Anteil an Verbindungen mit n = 4 mindestens 80%, bevorzugt mindestens 85%, besonders bevorzugt mindestens 90% und ganz besonders bevorzugt 95 bis 99% bezogen auf die Gesamtmenge an Verbindungen der Formel (I) beträgt.

2. Polysulfidmischung gemäß Anspruch 1, **dadurch gekennzeichnet, dass** der Anteil der Verbindungen der Formel (I) mit n = 3 weniger als 10%, bevorzugt weniger als 6%, besonders bevorzugt weniger als 4% und ganz besonders bevorzugt weniger als 2% bezogen auf die Gesamtmenge an Polysulfidverbindungen der Formel (I) beträgt.

3. Polysulfidmischung gemäß Anspruch 1 oder 2, **dadurch gekennzeichnet, dass** der Anteil der Verbindungen der Formel (I) mit n = 5 weniger als 10%, bevorzugt weniger als 6%, besonders bevorzugt weniger als 4% und ganz besonders bevorzugt weniger als 3% bezogen auf die Gesamtmenge an Polysulfidverbindungen der Formel (I) beträgt.

4. Polysulfidmischung gemäß einem der Ansprüche 1 bis 3, **dadurch gekennzeichnet, dass** der Anteil an Verbindungen, die nicht der Formel (I) entsprechen, weniger als 10% besonders bevorzugt weniger als 3%, ganz besonders bevorzugt weniger als 1 % und meist bevorzugt weniger als 0,2% beträgt.

5. Polysulfidmischung gemäß einem der Ansprüche 1 bis 4, **dadurch gekennzeichnet, dass** R für Methyl steht.

6. Verfahren zur Herstellung von Polysulfidmischungen gemäß einem der Ansprüche 1 bis 5 durch Inkontaktbringen von 3-Mercaptopropionsäurealkylester mit S₂Cl₂ in einem inerten Medium, **dadurch gekennzeichnet, dass** als inertes Medium eine Polysulfidmischung gemäß einem der Ansprüche 1 bis 5 verwendet wird, oder dass ein anderes inertes Medium als eine Polysulfidmischung gemäß einem der Ansprüche 1 bis 5 verwendet wird und die Abtrennung dieses inerten Mediums bei einer Temperatur von nicht mehr als 45°C, bevorzugt nicht mehr als 40°C, besonders bevorzugt nicht mehr als 35°C und ganz besonders bevorzugt nicht mehr als 30°C durchgeführt wird.

7. Verfahren gemäß Anspruch 6, **dadurch gekennzeichnet, dass** das Inkontaktbringen von 3-Mercaptopropionsäurealkylester, insbesondere von 3-Mercaptopropionsäuremethylester mit S₂Cl₂ bei Temperaturen von 0°C bis 60°C, bevorzugt von 10 °C bis 45 °C, besonders bevorzugt von 15°C bis 35°C, ganz besonders bevorzugt von 20°C bis 30°C erfolgt.

8. Verfahren gemäß Anspruch 6 oder 7, **dadurch gekennzeichnet, dass** das entstehende HCl-Gas während der Umsetzung durch Durchleiten von Inertgas und/oder durch Anlegen von Vakuum ganz oder anteilig aus dem Ansatz entfernt wird.

9. Kautschukmischung enthaltend zumindest
- inen Kautschuk, und
- eine Polysulfidmischung gemäß einem der Ansprüche 1 bis 5.

10. Kautschukmischung gemäß Anspruch 9 enthaltend zusätzlich mindestens
- ein schwefelhaltiges Alkoxysilan, und
- einen kieselsäurebasierenden Füllstoff.

11. Kautschukmischung gemäß Anspruch 9 oder 10, **dadurch gekennzeichnet, dass** sie zusätzlich mindestens einen Vernetzer enthält.

12. Kautschukmischung gemäß einem der Ansprüche 9 bis 11, **dadurch gekennzeichnet, dass** sie als Kautschuk mindestens einen SBR-Kautschuk und mindestens einen BR-Kautschuk, vorzugsweise im Gewichtsverhältnis SBR-Kautschuk:BR-Kautschuk von 60:40 bis 90:10 enthält.

13. Kautschukmischung gemäß Anspruch 12, **dadurch gekennzeichnet, dass** sie zusätzlich mindestens einen NR-Kautschuk enthält, vorzugsweise in einem Gewichtsverhältnis SBR-Kautschuk zu BR-Kautschuk zu NR-Kautschuk von 60 bis 85 : 10 bis 35 : 5 bis 20.

14. Verfahren zur Herstellung von Kautschukmischungen gemäß einem der Ansprüche 9 bis 13, durch Mischen von mindestens jeweils einem Kautschuk und einer Polysulfidmischung gemäß einem der Ansprüche 1 bis 5.

15. Verfahren zur Herstellung von Vulkanisaten **dadurch gekennzeichnet, dass** man die Kautschukmischung gemäß einem der Ansprüche 9 bis 13 vulkanisiert, vorzugsweise bei Massetemperaturen von 100 bis 200°C, besonders bevorzugt von 130 bis 180°C.

16. Vulkanisate, erhältlich durch Vulkanisation der Kautschukmischungen gemäß einem der Ansprüche 9 bis 13.

17. Kautschukprodukte, insbesondere Reifen enthaltend ein oder mehrere Kautschukvulkanisate gemäß Anspruch 16.

18. Fahrzeug enthaltend Kautschukprodukt gemäß Anspruch 17.

19. Verwendung von Polysulfidmischungen gemäß einem der Ansprüche 1 bis 5 zur Herstellung von Kautschukmischungen und deren Vulkanisaten.

20. Additivzusammensetzung enthaltend jeweils mindestens ein schwelfelhaltiges Alkoxysilan, insbesondere Bis-(triethoxysilylpropyl)tetrasulfan, Bis-(triethoxysilylpropyl)disulfan, 3-(Triethoxysilyl)-1-propanthiol, polyetherfunktionalisiertes Mercaptosilan oder thioesterfunktionalisiertes Alkoxysilan und eine Polysulfidmischung gemäß einem der Ansprüche 1 bis 5.

21. Verwendung von Polysulfidmischungen gemäß einem der Ansprüche 1 bis 5 zur Herstellung von Additivzusammensetzungen gemäß Anspruch 20.

22. Verfahren zur Verringerung des Rollwiderstands von Reifen, **dadurch gekennzeichnet, dass** man einer nicht- oder teilvernetzten Kautschukmischung, welche als Ausgangsmaterial für zumindest Teile des Reifen dient, eine Polysulfidmischungen gemäß einem der Ansprüche 1 bis 5 beimengt und die Mischung anschließend vulkanisiert.
